# EUROPÄISCHE PATENTSCHRIFT

(11) **EP 2 792 305 B1**
(45) Veröffentlichungstag und Bekanntmachung des Hinweises auf die Patenterteilung: **04.08.2021**
(21) Anmeldenummer: 14153149.1
(22) Anmeldetag: 30.01.2014
(51) Int. Cl.: A61B 17/00, A61B 1/00, A61B 1/313, A61B 17/02, A61B 17/29, A61B 90/00

(54) **Medizinisches Instrument**
Medical instrument
Instrument médical

(30) Priorität: 18.04.2013 DE 102013103905
(43) Veröffentlichungstag der Anmeldung: 22.10.2014
(73) Patentinhaber: Karl Storz SE & Co. KG, 78532 Tuttlingen (DE)
(72) Erfinder: Hermle, Rainer, 78532 Tuttlingen (DE)

(56) Entgegenhaltungen:
- WO-A1-99/52418
- DE-A1- 19 906 260
- US-A- 5 681 262
- US-A- 5 902 233
- US-A- 6 139 489
- US-A1- 2001 012 942
- US-A1- 2003 220 547

## Beschreibung

Die vorliegende Erfindung betrifft ein medizinisches Instrument nach dem Oberbegriff von Anspruch 1.

Aus der Offenlegungsschrift DE 198 27 360 A1 ist ein medizinisches Instrument bekannt, das einen lang erstreckten Schaft aufweist, der am distalen Ende eine Spatelspitze aufweist, und in dessen proximalem Bereich ein seitlich abstehender Handgriff angeordnet ist. Der Handgriff ist so mit dem Schaft verbunden, dass eine von dem Handgriff abgewandte Außenseite des Instruments vom distalen bis zum proximalen Ende durchgehend eine im Wesentlichen gleichmäßige Fläche aufweist. Das bekannte Instrument weist ferner eine Endoskopoptik mit einer im Wesentlichen zur gleichen Seite wie der Handgriff bezüglich einer Längsmittelachse des Schafts schräg angeordneten Okularmuschel auf. Das bekannte medizinische Instrument ist zur endoskopischen Entnahme der Vena Saphena Magna bestimmt, wobei das Instrument durch einen Einschnitt im Kniebereich eingeführt und entlang der Vene bis zur Leistengegend bzw. bis in den Knöchelbereich nahezu parallel zur Hautoberfläche vorangeschoben wird. Durch den endoskopischen, unter Sichtkontrolle durchgeführten Eingriff ist eine besonders gewebeschonende Entnahme der Vena Saphena Magna möglich, wobei zudem nur eine einzige, relativ kurze Narbe verbleibt.

Auch bei Schilddrüsenoperationen sind endoskopische Verfahren bekannt. Bei der Methode EndoCATS (Endoscopic Cephalic Access Thyroid Surgery) erfolgt ein Schnitt in der behaarten Kopfhaut hinter dem Ohr. Durch natürliche Spalträume wird ein Retraktorspatel bis in die Schilddrüsenloge (*Spatium de Quervain*) vorgeschoben. Nach der Operation wird der Einschnitt durch das Nachwachsen der Haupthaare verdeckt, so dass die Narbe unsichtbar bleibt. Um den Spatel durch die natürlichen Spalträume zum eigentlichen Operationsgebiet zu führen, wird der Spatel bei der Vorschubbewegung je nach Lage und Richtung der nutzbaren Spalträume um die Längsachse des Instruments gedreht. Dadurch, dass das Instrument in seinem proximalen Endbereich dicht an der Kopfhaut anliegt, ist jedoch die Handhabung des Instruments zur Ausführung einer solchen Drehung erschwert und insbesondere der verfügbare Winkelbereich relativ eng begrenzt.

In diesem oder einem nahegelegenen Gebiet der Medizintechnik existieren bereits einige Patente und Patentanmeldungen.

Dabei zeigt DE 199 06 260 A1 einen Dissektionsretraktor zum Entnehmen insbesondere der Saphenavene, wobei der Retraktor in einer geschlossenen Einfuhr-Stellung mit übereinander liegenden Platten bzw. Schaftteilen mit bogenförmigem Querschnitt in eine Operationsstellung überführt werden kann, in der die bogenförmigen Schaftteile aufgefächert nebeneinander vorliegen und damit einen vergrößerten Arbeitsraum schaffen.

Weiters zeigen US 5 902 233 A und US 6 139 489 A und US 2003/220547 A1, die alle das Gebiet der Retraktoren betreffen, jeweils medizinische Instrumente, insbesondere Retraktoren, mit einem Schaft und distalem Spatel und proximalem Handgriff.

US 2001 /012942 A1 betrifft ebenso einen Retraktor und schlägt über die oben genannten genannten hinaus vor, den Schaft relativ zum Handgriff mittels Führung in einer kreisbogenförmigen Nut quer zur Schaft-Längsachse drehbar zu gestalten. Zudem sind hier Handgriff und Schaft voneinander lösbar.

Weitere Schriften betreffen als Anwendung das "Vena Saphena harvesting", nämlich die WO 99/52418 A1, beziehungsweise ein Endoskop, die US 5 681 262 A. Diese beiden Schriften zeigen ebenso Instrumente mit Schaft, Spatel und Handgriff. Bei der WO 99/52418 A1 ist überdies der Handgriff relativ zum Schaft drehbar.

Es ist Aufgabe der vorliegenden Erfindung, die Handhabbarkeit eines gattungsgemäßen medizinischen Instruments zu verbessern, insbesondere eines für die Methode EndoCATS bestimmten medizinischen Instruments.

Diese Aufgabe wird durch eine Vorrichtung gemäß Anspruch 1 gelöst.

Vorteilhafte Weiterbildungen der Erfindung ergeben sich aus den Unteransprüchen.

Ein erfindungsgemäßes medizinisches Instrument umfasst einen langerstreckten Schaft, der an einem distalen, d.h. benutzerfernen Ende einen Spatel trägt und in einem proximalen, d.h. benutzernahen Endbereich einen Schaftkopf aufweist, an dem ein seitlich abstehender Handgriff angeordnet ist. Der Spatel ist insbesondere abgeflacht oder löffeiförmig sowie atraumatisch ausgebildet, wodurch das Durchführen durch natürliche Spalträume erleichtert und mögliche Gewebeverletzungen minimiert werden. Der Schaftkopf kann mit dem Schaft fest verbunden sein oder einen integralen Bestandteil des Schafts bilden.

Der Handgriff ist insbesondere zum Umgreifen mit einer Hand ausgebildet und kann zur Verbesserung der Handhabung schräg gestellt sein. Der Schaft des medizinischen Instruments kann einen durchgehenden Kanal zum Einschieben einer Endoskopoptik aufweisen, wobei der Schaftkopf eine Kupplung zum Festlegen der Endoskopoptik im Schaft aufweisen kann. Ferner können weitere durchgehende Kanäle vorhanden sein, die etwa zum Spülen und/oder Saugen genutzt werden können.

Erfindungsgemäß ist der Schaft relativ zum Handgriff um eine zu einer Längsachse des Schafts im Wesentlichen parallele Drehachse drehbar. Die Drehachse kann mit einer Längsmittelachse des Schafts zusammenfallen oder gegenüber dieser versetzt sein.

Dadurch, dass der Schaft relativ zum Handgriff um eine Längsmittelachse des Schafts oder eine zu dieser parallelen Achse drehbar ist, wird es ermöglicht, den Schaft in einem größeren Winkelbereich zu drehen als es durch eine Drehung des Handgriffs allein bzw. eines starren Instruments möglich wäre. Dies ist insbesondere dann von Vorteil, wenn die Drehung des Handgriffs durch die Operationsbedingungen eingeschränkt ist, wie dies beim EndoCATS-Verfahren aufgrund des Anliegens des proximalen Endbereichs des Instruments am Kopf des Patienten der Fall ist. Hierdurch wird das Ausnutzen natürlicher Spalträume zum Vorschieben des Spatels bis zum Operationsgebiet erleichtert.

Gemäß einer bevorzugten Ausführungsform der Erfindung ist der Schaft gegen einen Reibungswiderstand relativ zum Handgriff drehbar gelagert. Der Reibungswiderstand umfasst insbesondere eine Haftreibung, die zum Einleiten einer Drehbewegung überwunden werden muss, kann aber auch eine Gleitreibung während einer Drehbewegung umfassen. Dadurch, dass die Drehbewegung des Schafts relativ zum Handgriff durch den Reibungswiderstand gebremst wird, wird die Handhabbarkeit des medizinischen Instruments weiter verbessert. Insbesondere wird es ermöglicht, durch eine Drehung des Handgriffs den Schaft auch gegen einen gewissen, vom Gewebe ausgeübten Widerstand oder gegen Drehmomente, die von ggf. am Schaft befestigten Versorgungsleitungen ausgeübt werden, gezielt um seine Längsachse oder um eine zur Längsachse des Schafts parallele Drehachse zu drehen. Hierdurch wird es weiter erleichtert, natürliche Spalträume zum Vorschieben des Spatels zu nutzen.

In vorteilhafter Weise wird der Reibungswiderstand durch einen federnd gelagerten Bremsstift oder durch ein federnd gelagertes Druckstück erzeugt. Hierdurch wird eine einfache und funktionssichere Anordnung geschaffen, wobei durch die Materialwahl und die Wahl der Federkraft ein für die Handhabung optimaler Reibungswiderstand gewährleistet werden können.

Vorzugsweise ist der Bremsstift bzw. das Druckstück dem Handgriff zugeordnet und wirkt auf eine dem Schaftkopf zugeordnete Reibfläche. Insbesondere kann der Bremsstift in einer Bohrung des Handgriffs geführt sein, in der ebenfalls eine Feder zur Erzeugung einer gewünschten Andrucckraft, mit der der Bremsstift an die Reibfläche angedrückt wird, angeordnet ist. Dadurch, dass der Bremsstift bzw. das Druckstück dem Handgriff zugeordnet ist, kann hierfür der im Handgriff vorhandene Bauraum genutzt werden und der Schaftkopf besonders kompakt ausgeführt werden.

Gemäß einer bevorzugten Ausführungsform der Erfindung ist der Schaft zu einer vom Handgriff abgewandten Außenseite des Instruments hin abgeflacht ausgebildet und bildet mit der vom Handgriff abgewandten Außenseite des Schaftkopfs eine im Wesentlichen vom Schaftkopf bis zum distalen Ende des Schafts durchgehende, zumindest weitgehend ebene Fläche, die insbesondere frei von Vorsprüngen und/oder Stufen ist. Die ebene Fläche kann auch leicht konkav ausgebildet sein und eine flache Rinne bilden, die vom proximalen Endbereich des Instruments bis zum distalen Ende des Schafts reicht. Insbesondere ist die im Wesentlichen ebene Fläche des Schaftkopfs und des Schafts gegenüber einer Mittelstellung des Handgriffs angeordnet. Dadurch, dass der Schaft und der Schaftkopf auf einer Außenseite des Instruments eine durchgehende, im Wesentlichen ebene Fläche bilden, wird es ermöglicht, den Schaft besonders dicht am Körper des Patienten durch eine Inzision einzuführen.

Vorzugsweise ist der Spatel löffelförmig ausgebildet und definiert einen Hohlraum innerhalb des Spatels. Mit dem Spatel kann ein weiterer, an den inneren Hohlraum anschließender Hohlraum im Gewebe offengehalten werden; zur Schaffung des Hohlraums ist in der Regel keine Gasinsufflation erforderlich. In dem Hohlraum können chirurgische Manipulationen, etwa die Entfernung eines Schilddrüsenlappens, unter endoskopischer Sicht vorgenommen werden, wofür eine Endoskopoptik durch einen im Schaft angeordneten Optikkanal und endoskopische Instrumente entlang des Schafts bis in den Bereich des Spatels vorgeschoben werden. Vorzugsweise ist der Hohlraum, den der Spatel definiert, zur vom Handgriff abgewandten Außenseite des Instruments hin offen, so dass die durch den Schaft und den Schaftkopf gebildete im Wesentlichen ebene Fläche auf der gleichen Seite des Instruments angeordnet ist wie der vom Spatel gebildete Hohlraum und in diesen übergeht. Hierdurch wird die Einführung und Bedienung endoskopischer Instrumente, die entlang des Schafts bis in den Hohlraum des Spatels oder einen durch den Spatel offengehaltenen Hohlraum eingeführt werden, erleichtert; dies gilt insbesondere bei einer rinnenförmigen Ausbildung der ebenen Fläche bzw. einer im Querschnitt flach nierenförmigen Ausbildung des Schafts.

Erfindungsgemäß ist der Handgriff relativ zum Schaftkopf in mindestens einer quer zur Längsachse des Schafts kreisbogenförmig verlaufenden Nut geführt. Der Mittelpunkt des Kreisbogens der mindestens einen kreisbogenförmigen Nut definiert hierbei die Drehachse, um die der Handgriff relativ zum Schaft gedreht werden kann. Die Drehachse kann beispielsweise die Mittellängsachse des Optikkanals sein, durch den eine Endoskopoptik in den Schaft eingeschoben werden kann, kann aber auch beispielsweise außerhalb dieser Mittellängsachse oder auch außerhalb des Schafts bzw. des Schaftkopfs liegen. Dadurch, dass der Handgriff relativ zum Schaftkopf in mindestens einer kreisbogenförmigen Nut geführt ist, wird eine für die Anwendung optimale Anordnung der Drehachse relativ zur Längsachse des Schafts ermöglicht.

Diese Ausbildung des medizinischen Instruments, bei der der Handgriff relativ zum Schaftkopf in mindestens einer quer zur Längsachse des Schafts kreisbogenförmig verlaufenden Nut geführt ist, ist besonders vorteilhaft, wenn der Schaft wie oben beschrieben zu einer vom Handgriff abgewandten Außenseite des Instruments hin abgeflacht ausgebildet ist und mit der vom Handgriff abgewandten Außenseite des Schaftkopfs eine im Wesentlichen vom Schaftkopf bis zum distalen Ende des Schafts durchgehende, zumindest weitgehend ebene oder leicht konkave Fläche bildet. In diesem Fall kann durch die Führung des Schafts in der kreisbogenförmig verlaufenden Nut vermieden werden, dass die Führung des Handgriffs in Richtung des Arbeitsraums auf der vom Handgriff abgewandten Außenseite des Schaftkopfs aufträgt. Hierdurch kann das medizinische Instrument besonders dicht am Körper des Patienten geführt werden, und die Einführung von endoskopischen Arbeitsinstrumenten parallel zum Schaft wird erleichtert.

Vorzugsweise weist die kreisbogenförmige Nut eine Hinterschneidung auf, durch die der Handgriff am Schaftkopf formschlüssig gehalten und geführt wird. Insbesondere kann die Nut in vorteilhafter Weise ein T-förmiges oder ein Schwalbenschwanz-Profil aufweisen. Hierdurch wird der besondere Vorteil erzielt, dass der Handgriff bei der Drehbewegung relativ zum Schaftkopf alleine durch den Eingriff in die Nut geführt wird, so dass der gesamte Querschnitt des Schafts und der Verlängerung des Schafts innerhalb des Schaftkopfs frei von Führungs- oder Halteelementen zum Führen bzw. Halten des Handgriff bleiben kann.

In besonders bevorzugter Weise weist der Schaftkopf die kreisbogenförmige Nut auf, und der Handgriff weist mindestens einen Vorsprung zum formschlüssigen Eingreifen in die Nut auf. Insbesondere ist der Vorsprung ebenfalls kreisbogenförmig und komplementär zum Querschnitt der Nut ausgebildet. Hierdurch wird auf besonders einfache Weise eine sichere Führung des Handgriffs zur Realisierung der Drehbarkeit des Handgriffs relativ zum Schaft ermöglicht.

Der Winkelbereich der Drehung zwischen Handgriff und Schaft wird durch mindestens einen, vorzugsweise zwei Anschlagstifte begrenzt, beispielsweise auf einen Winkelbereich von ± 35° bezüglich einer Mittelstellung. In weiter bevorzugter Weise sind der bzw. die Anschlagstifte am Schaftkopf angeordnet. Hierdurch wird die Handhabbarkeit des Instruments weiter verbessert, insbesondere wird es durch die Anordnung des bzw. der Anschlagstifte am Schaftkopf ermöglicht, dass ein Benutzer den Schaftkopf und damit den Schaft relativ zum Handgriff durch Greifen des bzw. der Anschlagstifte dreht.

Der Handgriff ist mit dem Schaftkopf lösbar verbunden. Hierfür ist mindestens ein Anschlagelement, insbesondere ein Anschlagstift, mit dem Schaftkopf lösbar verbunden und wird nach Entnahme des mindestens einen Anschlagstifts der Handgriff in der Nut über den durch den Anschlagstift begrenzten Bereich hinaus bewegt und dadurch vom Schaftkopf getrennt. Dadurch, dass der Handgriff von dem Schaftkopf gelöst werden kann, werden die Reinigung und Sterilisation des Instruments vereinfacht.

Vorzugsweise ist die Verbindung zwischen dem Handgriff und dem Schaftkopf nicht spielfrei. Dadurch werden die Montage und die Demontage des Handgriffs sowie die Handhabung des Instruments erleichtert.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen, welcher durch die Ansprüche definiert wird.

Weitere Aspekte der Erfindung ergeben sich aus der nachfolgenden Beschreibung eines bevorzugten Ausführungsbeispiels und der beigefügten Zeichnung. Es zeigen:
Fig. 1 ein medizinisches Instrument gemäß einem ersten Ausführungsbeispiel der Erfindung in einer perspektivischen Ansicht;
Fig. 2 das medizinische Instrument aus Fig. 1 in einer Seitenansicht;
Fig. 3 einen vergrößerten Teilschnitt des Instruments aus Fig. 1;
Fig. 4 das Instrument aus Fig. 1 in einer vergrößerten Vorderansicht mit aus der Mittelstellung heraus geschwenktem Handgriff;
Fig. 5 einen Zwischenschritt bei der Montage des Handgriffs der in Fig. 1 dargestellten Ausführungsform in einer perspektivischen Teilansicht;
Fig. 6 ein medizinisches Instrument gemäß einem zweiten Ausführungsbeispiel der Erfindung in einer perspektivischen Ansicht.

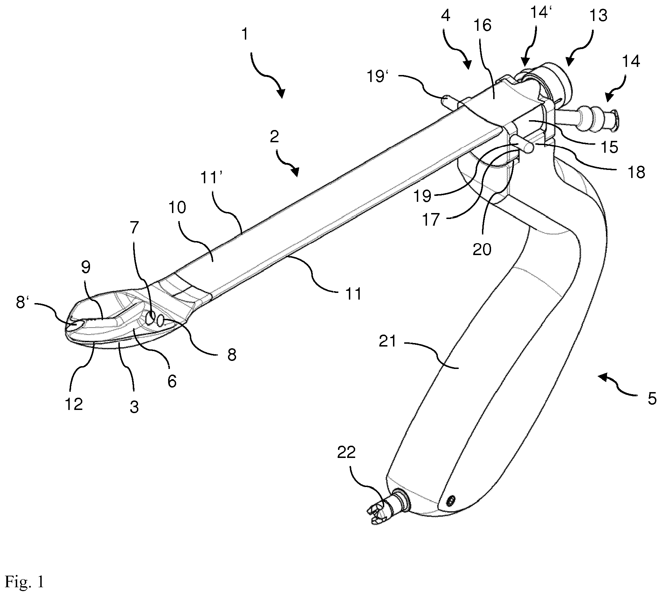

Das in Fig. 1 in einer perspektivischen Ansicht gezeigte medizinische Instrument 1 umfasst einen langerstreckten Schaft 2, an dessen distalem Ende ein löffelförmiger Spatel 3 und an dessen proximalem Ende ein Schaftkopf 4, an dem ein Handgriff 5 ansetzt, angeordnet sind. Der Spatel 3 ist näherungsweise in einer Verlängerung des Schafts 2 orientiert, jedoch leicht in der vom Handgriff 5 abgewandten Richtung, d.h. in der Darstellung der Figur 1 nach oben, abgewinkelt. Der löffelförmige Spatel 3 bildet in seinem Inneren einen Hohlraum 6 aus. In den Hohlraum 6 münden ein Optikkanal 7 und ein erster Spül-/Saugkanal 8. Ein weiterer Spül-/Saugkanal 8' ist durch ein abgewinkeltes Rohr 9 durch den Hohlraum 6 hindurchgeführt und mündet im distalen Endbereich des Spatels 3. Der Schaft 2 hat einen flach nierenförmigen Querschnitt und weist eine flach rinnenförmige Oberseite 10 auf. Die Kanten 11, 11' des Schafts 2 sowie die Kante 12 des Spatels 3 sind atraumatisch abgerundet.

In den Optikkanal 7 kann eine Endoskopoptik, die in Fig. 1 nicht dargestellt ist, eingesetzt werden. Diese kann ein in axialer Richtung gerichtetes oder ein abgewinkeltes Okular oder auch beispielsweise einen elektronischen Bildaufnehmer zur Aufnahme und Weiterleitung eines endoskopischen Bilds an eine Auswertungs- und Anzeigeeinrichtung aufweisen. Die Endoskopoptik ist vorzugsweise als Schrägblickoptik mit einer zur Längsachse um 30° bzw. 45° abgewinkelten Blickrichtung ausgebildet, die eine Beobachtung des vom Spatel 3 gebildeten Hohlraums 6 sowie eines ggf. durch den Spatel im Gewebe frei gehaltenen darüber liegenden Hohlraums oder auch eines natürlichen Hohlraums ermöglicht. In dem Hohlraum kann durch parallel zum Schaft 2 eingeführte Arbeitsinstrumente eine chirurgische Manipulation, etwa die Präparation und Abtrennung von Gewebe, erfolgen.

In seinem proximalen Endbereich ist der Schaft 2 fest mit dem Schaftkopf 4 verbunden, der eine Kupplung 13 sowie zwei Spül-/Sauganschlüsse 14, 14' trägt. Die Kupplung 13 ermöglicht die Fixierung einer durch den Optikkanal 7 in das Instrument 1 eingeschobenen starren Endoskopoptik. Die Kupplung 13 ist am proximalen Ende des durch den Schaft 2 und den Schaftkopf 4 hindurchgehenden Optikkanals 7 angeordnet. Die Spül-/Sauganschlüsse 14, 14' sind mit den Spül-/Saugkanälen 8, 8' verbunden und können beispielsweise in üblicher Weise als Luer-Lock ausgebildet sein. Der Optikkopf umfasst ferner einen Körper 15, der eine Oberseite 16 aufweist, die flach rinnenförmig ausgebildet ist und im Wesentlichen eine Fortsetzung der Oberseite 10 des Schafts 2 in proximaler Richtung bildet. Auf der dem Handgriff 5 gegenüberliegenden Außenseite des Instruments 1, die in der Darstellung der Figur 1 die Oberseite ist, entsteht dadurch eine vom proximalen Endbereich des Instruments 1 bis zum distalen Ende des Schafts 2 durchgehende, weitgehend glatte, von Vorsprüngen oder Stufen freie Fläche, die distalseitig in den Hohlraum 6 des löffelförmigen Spatels 3 mündet.

Der Körper 15 des Schaftkopfs 4 weist an seiner der Oberseite 16 abgewandten Unterseite eine kreisbogenförmige Nut 17 auf, in die ein komplementär geformter Vorsprung 18 des Handgriffs 5 eingesetzt ist. Wie unten näher erläutert wird, wird der Handgriff formschlüssig in der Nut 17 gehalten und ist durch Verschiebung des Vorsprungs 18 entlang der kreisbogenförmigen Nut 17 um eine zur Längsachse des Schafts 2 parallele Drehachse verschwenkbar. Zur Begrenzung des Schwenkwinkels trägt der Körper 15 zwei Anschlagzapfen 19, 19', an denen bei Erreichung eines Endwinkels eine jeweils entsprechende Schulter 20 des Handgriffs 5 anschlägt. Der Handgriff 5 ist insgesamt näherungsweise S-förmig ausgebildet und weist in seinem unteren Teil eine Grifffläche 21 auf, die mit in Fig. 1 nicht dargestellten Griffmulden ausgestattet sein kann. Am unteren Ende trägt der Handgriff 5 einen Haltezapfen 22, mit dem der Handgriff 5 und hierdurch das Instrument 1 mit einem üblichen Instrumentenhaltesystem verbunden werden kann.

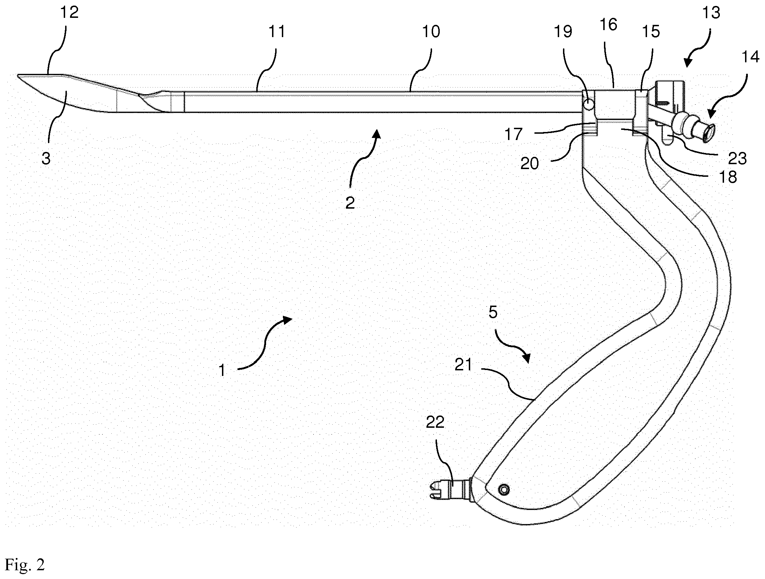

In der in Fig. 2 dargestellten Seitenansicht ist erkennbar, dass die Oberseite 10 des Schafts nahezu ohne Stufe in die Oberseite 16 des Körpers 15 des Schaftkopfs 4 übergeht. Der Spatel 3 ist gegenüber dem Schaft 2 leicht nach oben abgewinkelt. In Fig. 2 ist ferner ein Verriegelungsstift 23 sichtbar, durch dessen Betätigung eine in das Instrument 1 eingesetzte Endoskopoptik mit Hilfe der Kupplung 13 verriegelt und wieder entriegelt werden kann.

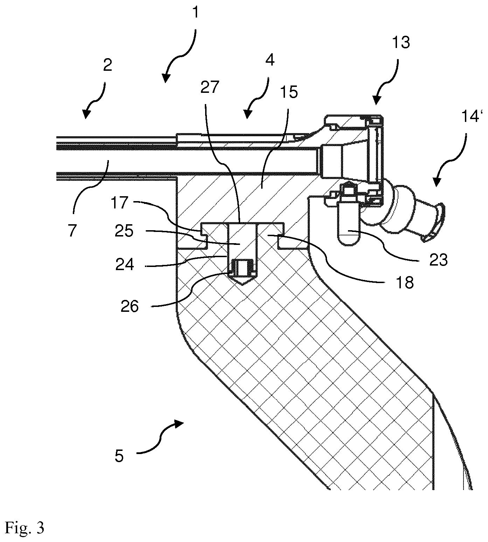

Wie in Fig. 3 in einem vergrößerten Längsschnitt dargestellt, ist die Nut 17 mit einer Hinterschneidung ausgebildet, in die der komplementär T-förmig ausgebildete Vorsprung 18 des Handgriffs 5 eingreift. Innerhalb des Handgriffs 5 ist in einer Bohrung 24 ein Bremsstift 25 verschiebbar gelagert, der durch eine Feder 26 gegen eine Reibfläche 27 an der Basis der T-förmigen Nut 17 des Körpers 15 des Schaftkopfs 4 gedrückt wird. In Fig. 3 ist nur ein Spül-/Sauganschluss 14' gezeigt. In Fig. 3 ist ferner der durch den Schaft 2 und den Schaftkopf 4 durchgehende Optikkanal 7 zu erkennen, der proximalseitig in der Kupplung 13 endet.

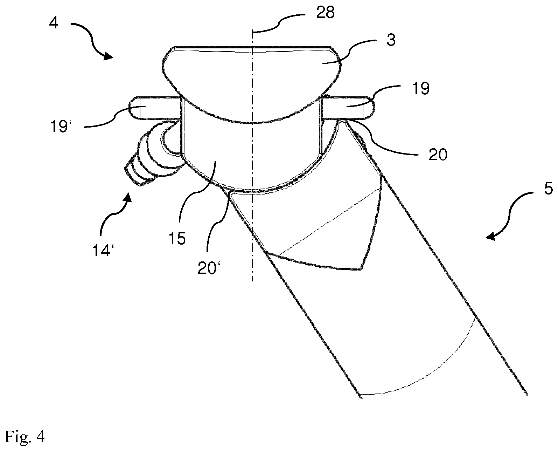

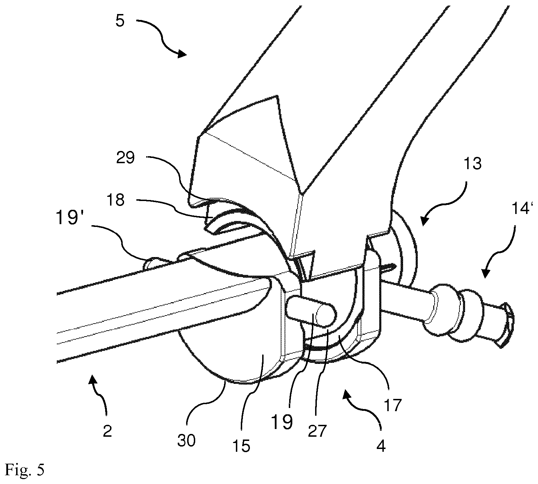

Fig. 4 zeigt eine aus distaler Richtung gesehene Teilansicht des Instruments 1 mit dem Spatel 3 und dem Körper 15 des Optikkopfs 4. Der Schaft 2 ist durch den Spatel 3 verdeckt. In Fig. 4 ist der Handgriff 5 in einer abgewinkelten Stellung gezeigt; diese ist eine Endstellung der Abwinkelung, bei der eine Schulter 20 in Anschlag mit einem Anschlagzapfen 19 steht. In der Mittelstellung fällt die Mittelebene des Handgriffs 5 mit der Längsmittelebene 28 des Instruments zusammen; bei der Maximalauslenkung in anderer Richtung schlägt die andere Schulter 20' am anderen Anschlagzapfen 19' an (in Fig. 4 nicht gezeigt). Die mögliche Drehbewegung des Handgriffs 5 wird hierdurch beispielsweise auf einen symmetrisch zur Mittelstellung angeordneten Winkelbereich von etwa ± 35° eingeschränkt. Die Drehachse, die durch den Mittelpunkt der kreisbogenförmigen Nut 17 definiert wird, fällt beispielsweise mit der Längsmittelachse des Optikkanals 7 zusammen (s. Fig. 1). Die T-Nut 17 kann sich an ihrer Basis über einen Winkelbereich von ca. 150° erstrecken (s. auch Fig. 5).

Wie in Fig. 5 gezeigt, kann der Handgriff 5 mit dem Schaftkopf 4 dadurch verbunden werden, dass der Handgriff 5 mit seinem T-förmigen Vorsprung 18, der wie die Nut 17 kreisbogenförmig ausgebildet ist, in die T-förmige Nut 17 eingeführt wird. Auch eine angrenzende Außenkontur 29 des Handgriffs 5 ist kreisbogenförmig ausgebildet und liegt an einer entsprechenden Außenkontur 30 des Körpers 15 des Schaftkopfs 4 an. Der Bremsstift 25 (s. Fig. 3) wird beim Ansetzen des Handgriffs 5 mit einem Finger oder einem Hilfswerkzeug in die Bohrung 24 eingedrückt, bis der Vorsprung 18 des Handgriffs 5 so weit in die Nut 17 eingeführt ist, dass der Bremsstift 25 durch Kontakt mit der Reibfläche 27 in der Bohrung 24 gehalten wird. Zum Verbinden des Handgriffs 5 mit dem Schaftkopf 4 wird einer der Anschlagstifte beziehungsweise Anschlagzapfen 19, 19' vom Körper 15 des Schaftkopfs 4 gelöst und, nachdem der Vorsprung 18 in die Nut 17 eingeführt worden ist und der Handgriff 5 nahezu seine Mittelstellung erreicht hat, wieder eingesetzt (in Fig. 5 nicht dargestellt). Erfindungsgemäß sind die Anschlagstifte beziehungsweise Anschlagzapfen 19, 19' in den Körper 15 des Schaftkopfs 4 einschraubbar und aus diesem wieder entnehmbar ausgebildet, um eine lösbare Verbindung des Handgriffs 5 mit dem Schaftkopf 4 zu schaffen. Sofern andererseits, in einem nicht erfindungsgemäßen Beispiel, die Verbindung zwischen dem Handgriff 5 und dem Schaftkopf 4 nicht durch einen Benutzer lösbar sein soll, sind die Anschlagstifte beziehungsweise Anschlagzapfen 19, 19' fest mit dem Schaftkopf 4 verbunden, beispielsweise durch Verklebung des Schraubgewindes.

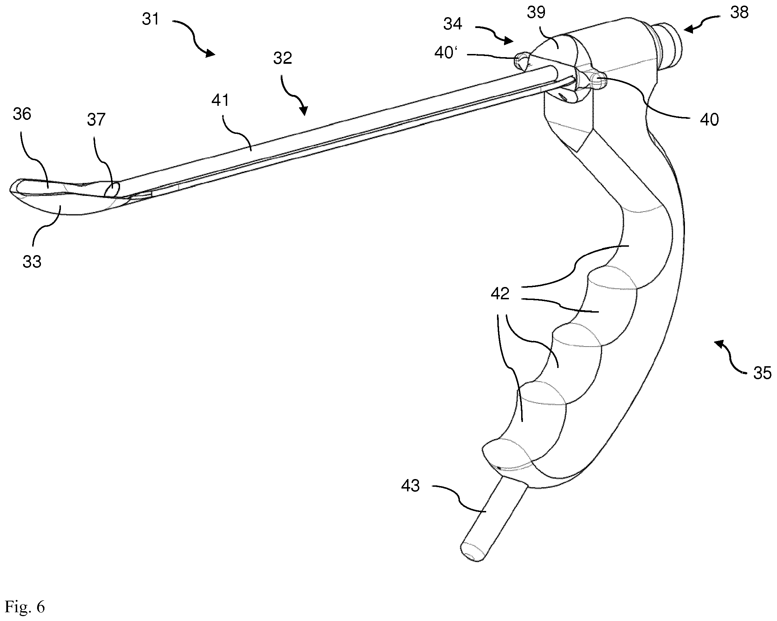

Gemäß dem in Fig. 6 dargestellten zweiten Ausführungsbeispiel der Erfindung weist ein medizinisches Instrument 31 einen lang erstreckten Schaft 32, einen am distalen Ende des Schafts 32 angeordneten löffelförmigen Spatel 33, einen Schaftkopf 34 am proximalen Ende des Schafts 32 und einen seitlich abstehenden, am Schaftkopf 34 ansetzenden Handgriff 35 auf. Der Schaft 32 ist rinnenförmig ausgebildet, wobei innerhalb der Rinne ein Rohr 41 mit einem durchgehenden Optikkanal 37 angeordnet ist, der in einer in den von dem löffelförmigen Spatel 33 gebildeten Hohlraum 36 mündenden Öffnung endet. Am proximalen Ende des Schafts setzt sich der Optikkanal 37 durch den Schaftkopf 34 hindurch fort und endet in einer Kupplung 38, mit deren Hilfe eine in den Optikkanal 37 eingeschobene Endoskopoptik am medizinischen Instrument 31 fixiert werden kann. Der Schaftkopf 34 umfasst eine relativ zum Schaft 32 feststehende Stirnwand 39, die zwei seitlich abstehende, fest mit der Stirnwand 39 verbundene Haltezapfen 40, 40' trägt. Proximalseitig der Stirnwand 39 umgreift der Handgriff einen als Drehlager ausgebildeten Abschnitt des Schaftkopfs 34. Der Handgriff 35 ist näherungsweise S-förmig gekrümmt und weist im unteren, schräg zum distalen Ende des Instruments 31 abgewinkelten Bereich eine Mehrzahl an Griffmulden 42 auf, die ein ergonomisches und sicheres Halten des Handgriffs 35 mit einer Hand des Benutzers erleichtern. Am unteren Ende trägt der Handgriff 35 einen Haltezapfen 43, mit dem der Handgriff 35 und damit das medizinische Instrument 31 in ein übliches Haltesystem eingesetzt und dort gehalten werden kann. Der Handgriff 35 ist um die Mittellängsachse des Optikkanals 37 relativ zum Schaft 32 bzw. relativ zum Schaftkopf 34, der fest mit dem Schaft 32 verbunden ist, drehbar. Zum Ausführen der Drehung kann ein Benutzer den Schaftkopf 34 an den Haltezapfen 40, 40' halten und den Handgriff 35 gegen den Schaftkopf 34 drehen. Der Drehwinkel kann durch einen nicht dargestellten Anschlag begrenzt sein. Im Übrigen ist das medizinische Instrument 31 wie zum ersten Ausführungsbeispiel beschrieben ausgebildet und kann insbesondere einen oder mehrere nicht dargestellte Spül-/Saugkanäle mit entsprechenden Anschlüssen aufweisen.

Vor der Verwendung des in den Figuren gezeigten medizinischen Instruments 1,31 wird durch den Optikkanal 7, 37 eine Endoskopoptik vom proximalen Ende her bis zum Erreichen der distalen Öffnung des Optikkanals 7, 37 oder geringfügig darüber hinaus eingeführt, so dass eine endoskopische Beobachtung zumindest des vom Spatel 3, 33 gebildeten Hohlraums 6, 36 möglich ist. Die eingeführte Endoskopoptik wird mit der Kupplung 13, 38 verriegelt und mit beispielsweise einem Leitkabel zur Einkopplung von durch eine externe Lichtquelle erzeugtem Beleuchtungslicht verbunden. Ggf. können zu diesem oder auch zu einem späteren Zeitpunkt Saug- bzw. Spülleitungen an die Saug-/Spülanschlüsse 14, 14' angeschlossen werden.

Zum Erreichen des Operationsgebiets bei einer Schilddrüsenoperation nach der EndoCATS-Methode wird der Handgriff 5, 35 des medizinischen Instruments 1,31 vom Benutzer umfasst und der Spatel 3, 33 mit dem Schaft 2, 32 durch einen hinter dem Ohr des Patienten ausgeführten Schnitt eingeführt. Unter endoskopischer Sicht, die durch ein Okular der Endoskopoptik oder auch durch eine Darstellung des endoskopischen Bilds auf einem Bildschirm vermittelt wird, wird der Spatel 3, 33 durch natürliche Spalträume hindurchgeführt. Sofern je nach Ausrichtung der betreffenden Spalträume eine Drehung des Spatels 3, 33 und des Schafts 2, 32 um die Längsachse erforderlich ist, kann diese durch entsprechende Drehung des Handgriffs 5, 35 ausgeführt werden. Um den möglichen Drehwinkelbereich zu erweitern oder auch um eine bessere Handhabung zu ermöglichen, kann stattdessen oder zusätzlich eine Drehbewegung zwischen dem Schaftkopf 4, 34 und dem Handgriff 5, 35 genutzt werden. Hierfür greift der Benutzer an den Anschlagzapfen 19, 19' bzw. den Haltezapfen 40, 40' an und dreht den Schaftkopf 4, 34 und damit den Schaft 2, 32 mit dem Spatel 3, 33 um eine zur Längsachse des Schafts 2, 32 parallele Drehachse relativ zum Handgriff 5, 35, wobei der durch den Bremsstift 25, der auf die Reibfläche 27 drückt, erzeugte Reibungswiderstand überwunden werden muss. Dabei kann die Drehachse, die beispielsweise durch die kreisbogenförmige Nut 17 definiert wird, so gelagert sein, dass sie im Wesentlichen mit der Längsachse einer in den Optikkanal 7, 37 eingesetzten Endoskopoptik übereinstimmt. Hierdurch wird die endoskopische Beobachtung beim Einführen und Rotieren des Schafts 2, 32 erleichtert.

Ist der Spatel 3, 33 bis zum eigentlichen Operationsgebiet, beispielsweise der Schilddrüsenloge (*Spatium de Quervain*) vorgeschoben worden, so werden endoskopische Arbeitsinstrumente parallel zum Schaft 2, 32 in das Operationsgebiet vorgeschoben. Dies wird bei dem oben beschriebenen ersten Ausführungsbeispiel der Erfindung dadurch erleichtert, dass die Oberseiten 10, 16 des Schafts 2 und des Körpers 15 des Schaftkopfes 4 eine im Wesentlichen durchgehende ebene Fläche oder eine flache Rinne bilden, an bzw. in der die endoskopischen Arbeitsinstrumente vorgeschoben werden können. Mit den Arbeitsinstrumenten können in dem vom Spatel 3,33 gebildeten inneren Hohlraum 6, 36 oder einen vom Spatel 3, 33 durch Druck auf umgebendes Gewebe zusätzlich offengehaltenen erweiterten Hohlraum chirurgische Manipulationen unter endoskopischer Sicht vorgenommen werden. So kann beispielsweise ein Schilddrüsenlappen freipräpariert und durch Herausziehen des Schafts 2, 32 mit dem Spatel 3, 33 im Hohlraum 6,36 des Spatels 3, 33 gehalten und dadurch im Ganzen geborgen werden. Auch dies kann durch die Ausbildung der Oberseiten 10, 16 des Schafts 2 und des Körpers 15 des Schaftkopfes 4 als im Wesentlichen durchgehende ebene Fläche oder flache Rinne, gemäß dem ersten Ausführungsbeispiel der Erfindung, erleichtert werden.

Eine Spülung des Operationsgebiets kann durch die Spül-/Saugkanäle 8, 8' bewirkt werden, wobei der in der Nähe des Optikkanals 7 mündende Spül-/Saugkanal 8' auch eine Spülung des Fensters der eingeführten Endoskopoptik ermöglicht. Die Spülflüssigkeit sowie ggf. Körperflüssigkeiten und/oder Rauchgase, die bei der Verwendung elektrochirurgischer Arbeitsinstrumente entstehen können, können durch die Spül-/Saugkanäle 8, 8' ebenfalls abgesaugt werden.

Der Übersichtlichkeit halber sind nicht in allen Figuren alle Bezugszeichen dargestellt. Zu einer Figur nicht erläuterte Bezugszeichen haben die gleiche Bedeutung wie in den übrigen Figuren.

### Bezugszeichenliste

- 1: Medizinisches Instrument
- 2: Schaft
- 3: Spatel
- 4: Schaftkopf
- 5: Handgriff
- 6: Hohlraum
- 7: Optikkanal
- 8, 8': Spül-/Saugkanal
- 9: Rohr
- 10: Oberseite
- 11, 11': Kante
- 12: Kante
- 13: Kupplung
- 14, 14': Spül-/Sauganschluss
- 15: Körper
- 16: Oberseite
- 17: Nut
- 18: Vorsprung
- 19, 19': Anschlagzapfen
- 20, 20': Schulter
- 21: Grifffläche
- 22: Haltezapfen
- 23: Verriegelungsstift
- 24: Bohrung
- 25: Bremsstift
- 26: Feder
- 27: Reibfläche
- 28: Längsmittelebene
- 29: Außenkontur
- 30: Außenkontur
- 31: Medizinisches Instrument
- 32: Schaft
- 33: Spatel
- 34: Schaftkopf
- 35: Handgriff
- 36: Hohlraum
- 37: Öffnung
- 38: Kupplung
- 39: Stirnwand
- 40, 40': Haltezapfen
- 41: Rohr
- 42: Griffmulde
- 43: Haltezapfen

## Patentansprüche

1. Medizinisches Instrument mit einem langerstreckten Schaft (2, 32), der an einem distalen Ende einen Spatel (3, 33) trägt und in einem proximalen Endbereich einen Schaftkopf (4, 34) aufweist, an dem ein seitlich abstehender Handgriff (5, 35) angeordnet ist,
wobei der Schaft (2, 32) relativ zum Handgriff (5, 35) um eine zu einer Längsachse des Schafts (2, 32) parallele Drehachse drehbar ist,
wobei der Handgriff (5) relativ zum Schaftkopf (4) in einer quer zur Längsachse des Schafts (2) verlaufenden kreisbogenförmigen Nut (17) geführt ist,
wobei der Handgriff (5, 35) lösbar mit dem Schaftkopf (4, 34) verbunden ist,
**dadurch gekennzeichnet, dass**
zur Begrenzung eines Winkelbereichs der Drehung zwischen dem Handgriff (5, 35) und dem Schaftkopf (4, 34) mindestens ein Anschlagstift (19, 19') angeordnet ist, wobei nach Entnahme des Anschlagstifts (19, 19') der Handgriff (5, 35) in der Nut (17) über den durch den Anschlagstift (19, 19') begrenzten Bereich hinaus bewegt und dadurch vom Schaftkopf (4, 34) getrennt werden kann.

2. Medizinisches Instrument nach Anspruch 1, **dadurch gekennzeichnet, dass** der Schaft (2, 32) gegen einen Reibungswiderstand drehbar ist.

3. Medizinisches Instrument nach Anspruch 2, **dadurch gekennzeichnet, dass** zur Erzeugung des Reibungswiderstands ein federnd gelagerter Bremsstift (25) oder ein federnd gelagertes Druckstück vorgesehen ist.

4. Medizinisches Instrument nach Anspruch 3, **dadurch gekennzeichnet, dass** der Bremsstift (25) bzw. das Druckstück dem Handgriff (5, 35) zugeordnet ist und auf eine Reibfläche (27) des Schaftkopfs (4, 34) wirkt.

5. Medizinisches Instrument nach einem der vorhergehenden Ansprüche, **dadurch gekennzeichnet, dass** der Schaft (2) an einer Oberseite (10) abgeflacht ist und mit einer Oberseite (16) des Schaftkopfs (4) eine im Wesentlichen durchgehende Fläche bildet.

6. Medizinisches Instrument nach einem der vorhergehenden Ansprüche, **dadurch gekennzeichnet, dass** die Nut ein T-förmiges oder ein schwalbenschwanzförmiges Profil aufweist.

7. Medizinisches Instrument nach einem der vorhergehenden Ansprüche, **dadurch gekennzeichnet, dass** die Nut (17) in einem Körper (15) des Schaftkopfs (4) angeordnet ist und dass der Handgriff (5) mindestens einen komplementär zur Nut (17) ausgebildeten Vorsprung (18) zum formschlüssigen Eingreifen in die Nut (17) aufweist.

## Claims

1. Medical instrument having an elongate shaft (2, 32) which bears a spatula (3, 33) at a distal end and in a proximal end region has a shaft head (4, 34) on which a laterally projecting handle (5, 35) is arranged, wherein the shaft (2, 32) is rotatable relative to the handle (5, 35) about an axis of rotation parallel to a longitudinal axis of the shaft (2, 32),
wherein the handle (5) is guided relative to the shaft head (4) in a circular arc-shaped groove (17) running transversely with respect to the longitudinal axis of the shaft (2),
wherein the handle (5, 35) is releasably connected to the shaft head (4, 34),
**characterized in that**
at least one stop pin (19, 19') is arranged in order to delimit an angular region of the rotation between the handle (5, 35) and the shaft head (4, 34), wherein, after removal of the stop pin (19, 19'), the handle (5, 35) in the groove (17) can be moved beyond the region delimited by the stop pin (19, 19') and thus separated from the shaft head (4, 34).

2. Medical instrument according to Claim 1, **characterized in that** the shaft (2, 32) is rotatable against a frictional resistance.

3. Medical instrument according to Claim 2, **characterized in that,** to generate the frictional resistance, a resiliently mounted braking pin (25) or a resiliently mounted pressure piece is provided.

4. Medical instrument according to Claim 3, **characterized in that** the braking pin (25) or the pressure piece is assigned to the handle (5, 35) and acts on a friction surface (27) of the shaft head (4, 34).

5. Medical instrument according to one of the preceding claims, **characterized in that** the shaft (2) is flattened on an upper side (10) and forms a substantially continuous surface with an upper side (16) of the shaft head (4).

6. Medical instrument according to one of the preceding claims, **characterized in that** the groove has a T-shaped or a dovetail-shaped profile.

7. Medical instrument according to one of the preceding claims, **characterized in that** the groove (17) is arranged in a body (15) of the shaft head (4), and **in that** the handle (5) has at least one projection (18) which is formed in a complementary manner to the groove (17) and intended for form-fitting engagement in the groove (17).

## Revendications

1. Instrument médical comprenant une tige allongée (2, 32) qui porte, à une extrémité distale, une spatule (3, 33) et qui présente dans une région d'extrémité proximale, une tête de tige (4, 34) au niveau de laquelle est disposée une poignée faisant saillie latéralement (5, 35),
la tige (2, 32) pouvant tourner par rapport à la poignée (5, 35) autour d'un axe de rotation parallèle à un axe longitudinal de la tige (2, 32),
la poignée (5) étant guidée par rapport à la tête de tige (4) dans une rainure (17) en forme d'arc de cercle s'étendant transversalement à l'axe longitudinal de la tige (2),
la poignée (5, 35) étant raccordée de manière amovible à la tête de tige (4, 34),
**caractérisé en ce que**
pour limiter une plage angulaire de rotation entre la poignée (5, 35) et la tête de tige (4, 34), il est prévu au moins une goupille de butée (19, 19')
après l'enlèvement de la goupille de butée (19, 19'), la poignée (5, 35) dans la rainure (17) étant déplacée au-delà de la région limitée par la goupille de butée (19, 19') et pouvant de ce fait être séparée de la tête de tige (4, 34).

2. Instrument médical selon la revendication 1, **caractérisé en ce que** la tige (2, 32) peut tourner à l'encontre d'une résistance de frottement.

3. Instrument médical selon la revendication 2, **caractérisé en ce que** pour reproduire la résistance de frottement, il est prévu une goupille de frein supportée sur ressort (25) ou une pièce de pression supportée sur ressort.

4. Instrument médical selon la revendication 3, **caractérisé en ce que** la goupille de frein (25) ou la pièce de pression est associée à la poignée (5, 35) et agit sur une surface de frottement (27) de la tête de tige (4, 34).

5. Instrument médical selon l'une quelconque des revendications précédentes, **caractérisé en ce que** la tige (2) est aplatie au niveau d'un côté supérieur (10) et forme avec un côté supérieur (16) de la tête de tige (4) une surface sensiblement continue.

6. Instrument médical selon l'une quelconque des revendications précédentes, **caractérisé en ce que** la rainure présente un profil en forme de T ou en forme de queue d'aronde.

7. Instrument médical selon l'une quelconque des revendications précédentes, **caractérisé en ce que** la rainure (17) est disposée dans un corps (15) de la tête de tige (4) et **en ce que** la poignée (5) présente au moins une saillie (18) réalisée de manière complémentaire à la rainure (17) pour l'engagement par correspondance de formes dans la rainure (17).
